# EUROPÄISCHE PATENTANMELDUNG

(11) **EP 1 754 771 A2**
(43) Veröffentlichungstag der Anmeldung: **21.02.2007**
(21) Anmeldenummer: 06015891.2
(22) Anmeldetag: 31.07.2006
(51) Int. Cl.: C10J 3/00

(54) **Verfahren zur Verwertung von biologischen Materialien**

(30) Priorität: 01.08.2005 DE 102005036573
(71) Anmelder: Stadtwerke Düsseldorf AG, 40233 Düsseldorf (DE)
(72) Erfinder: Damm, Udo, 44869 Bochum (DE); Erich, Egon, 47475 Kamp-Lintfort (DE)
(74) Vertreter: Bergen, Klaus

(57) **Zusammenfassung**

Zur verbesserten Verwertung von biologischen und organischen Materialien, insbesondere zur Erzeugung von Gas, wird vorgeschlagen, mindestens zwei Biomasseverwertungsverfahren, insbesondere eine Biomassevergasung und eine Biomassevergärung, miteinander zu kombinieren.

## Beschreibung

Die Erfindung betrifft ein Verfahren zur Verwertung von biologischen und anderen organischen Materialien, insbesondere zur Erzeugung von Gas.

Biologisches Material, kurz Biomasse, wird unter anderem in Biomassevergasern verwertet, in denen Biogas erzeugt wird. Ein Beispiel für einen solchen Biomassevergaser ist der aus der DE 197 18 184 A1 bekannte Kombivergaser.

Bei der Vergasung von Biomasse wird ein Schwachgas mit einem Heizwert mit typischerweise nicht mehr als 6 MJ/m³ gewonnen, dessen Qualität abhängig von den dem Biomassevergaser zugeführten Einsatzstoffen ist. Mögliche Einsatzstoffe sind insbesondere Frischholz, Altholz, Grünschnitt, Papierschlämme etc. mit einer Wasserfeuchte bis zu ca. 40%. Neben Anteilen an höheren Kohlenwasserstoffen (bis zu 50g/m³ Teere bei z.B. einfachen Gleichstromvergasern) und Staub enthält das Produktgas Produktwasser als Begleitkomponente. Die Hauptbestandteile des Gases, Stickstoff, Kohlenmonoxid, Kohlendioxid, Wasserstoff und Methan können in einem Gasmotor oder einer Gasturbine zur Erzeugung von elektrischer Energie genutzt werden.

Typischerweise enthält das Produktgas aus einer Biomassevergaseranlage ca.
H₂: 11,5 - 13,4Vol-%
CO: 21,6 22,4
CH₄: 0,9 - 3,1
CO₂: 9,9 - 10,4
N₂: 49,9 - 56,1

Selbst wenn die genannten Kombivergaser gegenüber anderen Vergasern wie Flachbettvergasern ein vergleichsweise teerarmes Produktgas erzeugen, ist auch bei diesem Vergasertyp beim An- und Abfahren der Vergasungsanlage je nach Einsatzmaterial mit einem besonders erhöhten Anteil an Teer und Staub im Produktgas zu rechnen. Der Teer verklebt mit dem Staub und setzt sich in Leitungen sowie Ventilen ab. Da der Staub, in der Regel alkalisch ist, abrasive Eigenschaften hat und zusätzlich auf Grund der Alkalimetalle einen relativ niedrige Schmelzpunkt aufweist, kann es zu zusätzlichen Problemen im Motor (wie Aufschmelzen der Asche im Verbrennungsraum) kommen.

Die hohen Anforderungen an Gasmotoren und Gasturbinen zur Erzeugung elektrischer Energie bezüglich des Teer- und Staubgehalts verhindern somit regelmäßig die direkte Nutzung des in einem Vergaser erzeugten Produktgases. Die Reinheitsbedingungen zur Nutzung des Gases in einem Gasmotor oder Gasturbine sind:
Teer <50mg/m3
Staub <50mg/m3

Bei der notwendigen Gaskühlung und Gasreinigung vor der Nutzung in einem Motor oder in einer Gasturbine fallen Kondensate an, die aus wasserunlöslichen Teerkomponenten, Feststoffen und aus Wasser, das mit einer Vielzahl löslicher, organischer Substanzen verunreinigt ist, bestehen. Das sogenannte Prozesswasser muss, je nach Gehalt an gelösten Kohlenwasserstoffen und festen Partikeln, vor der Abgabe, beispielsweise an eine kommunale Wasserentsorgung, einem Reinigungsschritt unterzogen werden. Eine wirtschaftliche Lösung der Wasseraufbereitung existiert bisher nicht.

Unter dem Begriff "Vergärung" ist der Abbau von biogenem Material durch Mikroorganismen in Abwesenheit von Sauerstoff, d.h. unter anaeroben Bedingungen zu verstehen. Mehrere Bakteriengruppen, welche sehr eng zusammenarbeiten, verwandeln biogenes Material in Biogas.

Die anaerobe Biomassevergärung wird hauptsächlich zur Entsorgung von feuchter Biomasse unter dem Aspekt der Brenngasgewinnung durchgeführt. Als Einsatzmaterial kommen die sogenannten Energiepflanzen (Gräser, z.B. Raigras, sortierter Hausmüll, Speisereste, Gülle, teilweise Industrieabfälle und Grünschnitt mit Holzbestandteilen aus der Wegepflege, Waldpflegeschnitt usw.) in Frage, zum Teil mit einem erheblichen Anteil an verholztem Material. Für die Vergärung eignen sich besonders feuchte Bioabfälle (Biotonne, Gülle u.ä.), weil sie beispielsweise gegenüber Pflanzenabfällen leichter abbaubar sind, mehr Nährstoffe enthalten und eine weichere Struktur besitzen.

Das Einsatzmaterial wird unter Luftabschluss in einem stehenden oder liegenden Faulbehälter durch Mikroorganismen bei ca. 55°C vergoren. Als Produkte erhält man ein insbesondere Methan enthaltendes Biogas und einen Rückstand, der nach Siebung z. B. als Dünger wieder ausgebracht werden kann. Der Siebrückstand enthält, je nach Einsatzmaterialien holzartige Reststoffe und schwer anaerob abbaubares organisches Material. Das Biogas hat typischerweise die folgende Zusammensetzung:
CH₄: 55-70 Vol.-%
CO₂: 27-44 Vol.-%
H₂: < 1 Vol.-%
H₂S: < 3 Vol.-%
Der Heizwert beträgt typischerweise 20 - 26 MJ/m³ bzw. 6-7 kWh/m³

Auch das Biogas kann, nach einer vorherigen Tropfenabscheidung (Demister) in einem Gasmotor verstromt werden. Der feste Siebrückstand, der die verholzte Biomasse enthält, muss üblicherweise entsorgt werden.

Beiden Verfahren ist gemeinsam, dass die Biomasse nur sehr unvollständig verwertet wird und insbesondere die nach der Vergasung bzw. Vergärung entstehenden Abfallprodukte, wie Prozesswasser und/oder Feststoffe, teilweise nur unwirtschaftlich entsorgt werden können.

Der Erfindung liegt die Aufgabe zugrunde, ein Verfahren der eingangs genannten Art zur Verfügung zu stellen, mit dem eine wirtschaftlichere Verwertung von biologischen Materialien möglich ist.

Gelöst wird diese Aufgabe mit den Merkmalen des Anspruchs 1.

Ein Kerngedanke der Erfindung besteht darin, dass durch die Kombination zweier oder mehrerer Biomasseverwertungsverfahren die bestehenden Nachteile der jeweils allein betriebenen Verfahren vermieden werden können, da die in einem der Verfahren anfallenden Abfallprodukte, wie beispielsweise Prozesswärme, Prozesswasser, Feststoffe, in mindestens einem der anderen Verfahren gewinnbringend genutzt werden können.

Zwei sich besonders gut ergänzende Verfahren sind die eingangs vorgestellten Verfahren zur Biomassevergasung und zur Biomassevergärung.

Aus dieser Kombination ergibt sich die besonders bevorzugte Möglichkeit, das Produktgas aus der Biomassevergasung einer flüssigen Phase (Maische) bei der Vergärung zuzuführen.

Es wurde nämlich überraschend festgestellt, dass das Produktgas aus dem Vergaser vollständig ohne weitere Konditionierung in den Reaktor einer Biomassevergärung gegeben werden kann. Eventuelle im Produktgas enthaltene höhermolekulare Kohlenwasserstoffe werden in der Maische im Vergärungsreaktor zu niederen Kohlenwasserstoffen, insbesondere zu Methan aufgeschlossen. Die heizwertreichen Komponenten des Produktgases werden nicht verändert (H₂, CO, CH₄). Aus der Vergärung der Biomasse einschließlich der zugeführten, höherwertige Kohlenwasserstoffe enthaltenden Inhaltsstoffe aus dem Produktgas entsteht als Brenngas Methan (CH₄) und Kohlendioxid (CO₂), also Verbindungen, die auch bei der Vergasung entstehen. Darüber hinaus wird im Produktgas enthaltener Staub herausgefiltert.

Ein weiterer Vorteil ergibt sich daraus, dass die Wärme des Produktgases zum Erhitzen der Maische im Vergärungsreaktor genutzt werden kann. Dadurch erhöht sich die Reaktionsgeschwindigkeit und damit die RaumZeitausbeute der Vergärung. Allerdings kann das Produktgas auch vor Eintritt in den Biomassevergärungsreaktor in einem Wärmetauscher teilweise oder ganz gekühlt werden, um Prozesswärme für eine anderweitige Nutzung zu gewinnen. Die Produktgasmenge aus dem Vergaser bzw. die Vergaserleistung und/oder die Produktgastemperatur können/kann dabei vorzugsweise so eingestellt werden, dass sich in der Vergärungsstufe die optimale Temperatur zur Vergärung einstellt.

Die meisten vorbekannten Biomassevergärungsanlagen werden üblicherweise im mesophilen Temperaturbereich (30° bis 40°C) betrieben, da eine Beheizung des Reaktors nicht wirtschaftlich ist. Durch das heiße Produktgas aus dem Vergaser lassen sich im Vergärungsreaktor auf einfache Weise Temperaturen bis 60°C bzw. 95°C einstellen und thermophile Bakterienstämme zur Vergärung einsetzen. Die erzielbare Gasmenge erhöht sich hierdurch, beispielsweise um ca. 20% bezogen auf die mesophile Vergärung bei 60°C Behandlungstemperatur und gleicher Verweilzeit im Vergärungsreaktor. Auch erfolgt bei höherer Temperatur ein schnellerer Abbau der organischen Stoffe. Die höhere Temperatur gewährleistet zudem eine bessere Hygienisierung der Maische, was für die Verwendung der Reststoffe aus der Vergärung als Rohdünger bzw. Humus wichtig ist. Kurze Verweilzeiten erbringen natürlich eine höhere Raum/Zeit-Ausbeute. Dadurch wird es auch möglich, den Biomassevergärungsreaktor kleiner auszuführen.

Prinzipiell sind durch das neue, kombinierte Verfahren auch höhere Vergärungstemperaturen realisierbar, die bisher an der wirtschaftlichen Bereitstellung der Reaktionswärme scheiterten. So ist es möglich, mit thermophilen Bakterienstämmen, z.B. vom Stamm der Thermogales, Behandlungstemperaturen von ca. 90°C und mit Bakterienstämmen wie Methanothermus fervidus Behandlungstemperaturen bis 97°C einzustellen, wodurch sich die Behandlungsdauer des Substrates unter Gewinnung von Methan deutlich verkürzt. Positive Nebeneffekte der höheren Behandlungstemperatur sind dabei die weitgehende Hygienisierung des Humus und die Geruchsminderung.

Des weiteren wurde überraschend festgestellt, dass auch das Produktwasser aus der Vergasung dem Reaktor zur Biomassevergärung zugeführt werden kann. So kann das Produktwasser als Anmaischwasser in der Vergärung dienen. Eventuell im Produktwasser enthaltene höhermolekulare Kohlenwasserstoffe können bei der Vergärung ebenfalls zu niederen Kohlenwasserstoffen, insbesondere zu Methan, aufgeschlossen werden.

Auch kann Flugstaub und die verbleibende Asche aus der Vergasungsstufe der Biomassevergärung zugeführt werden. Flugstaub und Asche haben einen erhöhten Alkalimetallgehalt. Aufgrund ihrer Alkalität (hohe Anteile an K, Na, Ca), gekennzeichnet auch durch den relativ hohen pH-Wert im Eluat, dienen sie als "Bodenverbesserer".

In umgekehrter Richtung können Reststoffe aus der Biomassevergärung wieder der Biomassevergasung zugeführt werden. Bevorzugt werden dafür feste Reststoffe, insbesondere Restholz und Humus, nach einer üblichen Behandlungsdauer der Biomassevergärung entnommen, der Humus davon abgetrennt und der verbleibende Rest der Biomassevergasung zugeführt. Dies kann kontinuierlich oder diskontinuierlich geschehen. Während der Humus angereichert ist und als Bodenersatzstoff weiter verwertet werden kann, können die verbleibenden, beispielsweise holzartige Bestandteile enthaltenden Feststoffe, die ein Kohlenstoffgerüst haben, durch die Biomassevergasung wirtschaftlich sinnvoll verwertet werden. Aus der Vergärung anfallendes Wasser ist biologisch aufbereitet und kann z.B. als Flüssigdünger ausgebracht werden.

Nachdem das durch die Kombination des Vergasungsprozesses und des Vergärungsprozesses erzeugte Gas weitestgehend die heizwertreichen Komponenten (H₂, CO, CH₄) enthält, kann es direkt einem Gasmotor oder einer Gasturbine zugeführt werden, ohne daß es einer aufwendigen Kühlung und Filterung bedarf. Das Gas sollte lediglich, wie es allgemein bei Vergärungsanlagen üblich ist, durch einen dem Gasmotor bzw. der Gasturbine vorgeschalteten Demister geführt werden, um Wassertröpfchen zurückzuhalten (zur Gewährleistung eines hohen Füllgrads des Motors mit Brenngas).

Somit ergibt sich aus der Kombination der Verfahren zur Biomassevergasung und Biomassevergärung eine Vielzahl von Vorteilen.

Der Vergärungsreaktor kann als biologische Kläranlage für das Produktgas aus dem Vergaser dienen, d.h. die übliche Gasreinigung und die Kühlung des Produktgases, die Abführung und die Klärung des Produktwassers etc., die einen Großteil der Investitionen und Betriebskosten ausmachen, können entfallen. Andererseits kann der Vergaser zur Entsorgung der nach der Vergärung zurückbleibenden, heizwertreichen Feststoffe dienen. Dadurch wird das System sehr flexibel bezüglich der Art und Menge der Einsatzstoffe.

Da das teerbehaftete Produktgas aus dem Vergaser, beispielsweise im bevorzugten Verhältnis von 1:10 bezogen auf die bei der Vergärung produzierte Gasmenge, zusammen mit eventuell noch vorhandenen festen Gasinhaltsstoffen (ca. 1-2 Gew.-% der zur Vergasung eingesetzten Feststoffmenge wie mineralischer Staub und Holzasche) in die flüssige Phase (Maische) der Vergärungsstufe eingebracht wird, muss die Betriebsweise des Vergasers nicht mehr an die Anforderung der Teerfreiheit im Produktgas angepaßt sein, sondern kann auf eine minimale Reststoffmenge (Asche) ausgelegt werden. Bevorzugt wird der Vergaser auf ca. 1-2% Asche gefahren. Dadurch wird der Energieinhalt des Einsatzmaterials besser ausgenutzt, und der energetische Wirkungsgrad der Vergaserstufe steigt.

Ein weiterer Vorteil ist, dass beide Teilanlagen auch weitgehend unabhängig bezüglich der Einsatzmengen, je nach Anfall des Einsatzmaterials betrieben werden können. Es können eine Vielzahl heizwertreicher Fraktionen zu Synthesegas umgewandelt werden, wie z.B. nachwachsende Rohstoffe, Reststoffe aus der Tierhaltung, Speisereste, Produktionsreste, Kunststoffabfälle, die im vorliegenden Fall als energetisch verwertbare Materialien ebenso als unter den Begriff Biomasse fallend angesehen werden, Klärschlämme, feste Brennstoffe, etc. Durch die weite Bandbreite möglicher Einsatzstoffe kann der resultierende Heizwert des erzeugten Gases vergleichmäßigt werden.

Ein weiterer Vorteil des erfindungsgemäßen Verfahrens ist, dass die Biomassevergärung unabhängig von der Biomassevergasung betrieben werden kann. Das erzeugte Gas dient ebenso wie beim kombinierten Verfahren als Feed für einen Gasmotor oder eine Gasturbine zur Erzeugung von elektrischer Energie. Umgekehrt kann das Produktgas aus dem Vergaser prinzipiell auch dann durch die Vergärungsstufe geführt und gereinigt werden, wenn diese sich im "Stand by"-Betrieb befindet, so dass auch die Biomassevergasung praktisch unabhängig von der Vergärungsstufe betrieben werden kann.

Das erfindungsgemäße Verfahren ermöglicht eine absolut von zu entsorgenden Reststoffen freie Verwertung der Einsatzstoffe. Wie bereits erwähnt, werden die aus der Vergasungsstufe in den Vergärungsreaktor eingeführten festen Partikel in den dort gebildeten Bodenersatzstoff (Humus) eingebunden. Das im Vergaser gebildete Produktwasser dient als Anmaischwasser, Teere werden umgewandelt.

Insgesamt wird durch das erfindungsgemäße Verfahren der Energiegehalt der Einsatzstoffe deutlich besser ausgenutzt, wodurch im Vergleich zu den bisher üblichen Einzelverfahren der Gesamtwirkungsgrad bzgl. Gewinnung von z.B. elektrischer Energie erheblich ansteigt.

Darüber hinaus können die kombinierten Verfahren ohne einen Wärmeeintrag von außen betrieben werden, vielmehr kann überschüssige Prozesswärme zur Beheizung von Gebäuden oder zu anderen Anwendungen ausgekoppelt werden.

Aus dem Vorstehenden wird deutlich, dass sich aus der Kombination der Verfahren gegenüber den Einzelverfahren erhebliche wirtschaftliche Vorteile ergeben.

Im Folgenden wird die Erfindung anhand der beigefügten Abbildungen, in denen unter anderem ein bevorzugtes Ausführungsbeispiel einer Anlage zum Betrieb des erfindungsgemäßen Verfahrens dargestellt ist, näher erläutert.

Es zeigen:
- Fig. 1: eine vereinfachte Prinzipskizze einer Anlage zur Durchführung des erfindungsgemäßen Verfahrens; und
- Fig. 2: eine schematische Darstellung einer Gesamtanlage zur Durch-führung des erfindungsgemäßen Verfahrens.

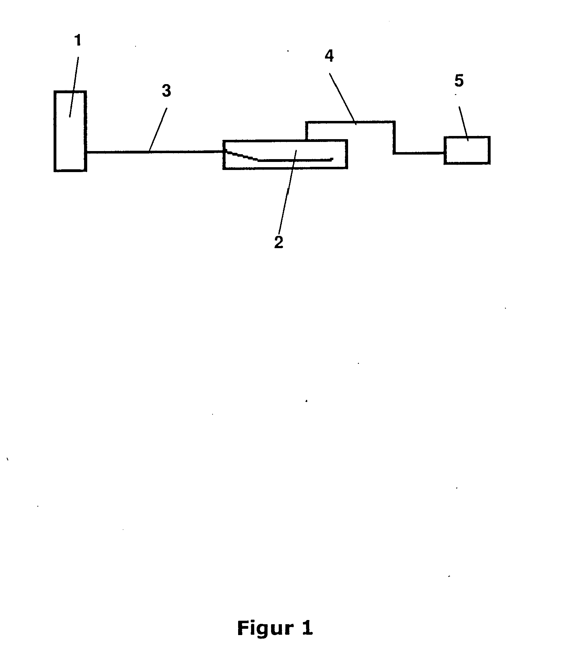

Die Skizze gemäß Figur 1 zeigt einen Vergaser 1, dem ein Vergärungsreaktor 2 nachgeschaltet ist. Das in dem Vergaser erzeugte Produktgas wird dem Vergärungsreaktor über eine Leitung 3 zugeführt. Das Produktgas wird in dem Vergärungsreaktor 2 gereinigt und in der oben beschriebenen Weise aufbereitet. Das aufbereitete Produktgas wird zusammen mit dem durch die Vergärung von Biomasse im Vergärungsreaktor erzeugten Gas über eine Leitung 4 einem beiden Reaktoren nachgeschalteten Gasmotor 5 zur Erzeugung elektrischer Energie zugeführt.

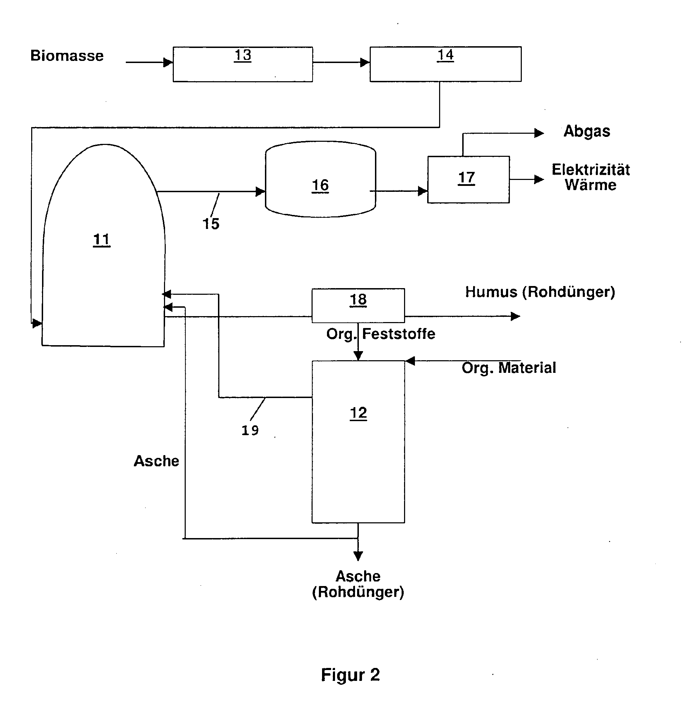

Die zentralen Komponenten der in Figur 2 dargestellten Gesamtanlage sind ebenfalls ein Vergärungsreaktor 11, beispielsweise ein stehender oder liegender Faulturm, sowie ein Vergaser 12.

Als Vergärungsreaktor kommen bevorzugt liegende Tankfermenter mit Rührwerk in Frage. Je nach Einsatzmaterial und Betriebsführung können mit den entsprechenden Bakterienstämmen die Hydrolyse- und Versäuerungsstufen der Vergärung in einem eigenen Gärungsreaktor durchgeführt werden, da die optimalen Parameter für die Hydrolyse/Versäuerung gegenüber der nachfolgenden Methangärung, insbesondere gegenüber einer thermophilen Methangärung, unterschiedlich sind (Zweistufenprozess). Vorzugsweise werden jedoch alle Vergärungsstufen mit dem geeigneten Bakterienmix in einem Vergärungsreaktor durchgeführt.

Dem Vergärungsreaktor 11 wird biologisches bzw. organisches Material zugeführt, das zuvor in einer Vorgrube 13 gesammelt und dessen Bestandteile später in einer Homogenisierungsstufe 14 zerkleinert und gemischt wurden, um bei der Vergärung einen möglichst gleichwertigen Heiz- bzw. Brennwert des erzeugten Gases zu erzielen.

Das im Vergärungsreaktor 11 erzeugte Gas wird über eine Leitung 15 einem Gasbehälter 16 zugeführt, über den ein Gasmotor 17 oder eine Gasturbine versorgt wird. Im Gasmotor wird Elektrizität und Wärme produziert, wobei die Wärme ausgekoppelt und genutzt, beispielsweise auch zur Beheizung des Vergärungsreaktors, werden kann.

Aus dem Vergärungsreaktor 11 können kontinuierlich oder diskontinuierlich Reststoffe abgeführt werden, die im Wesentlichen aus Humus und organischen Feststoffen bestehen. Der Humus wird von den organischen Feststoffen durch ein Sieb 18 getrennt, wobei der Humus als Bodenersatzstoff und Rohdünger einer weiteren Verwendung zugeführt werden kann.

Der Vergaser 12 wird mit zur Vergasung geeignetem organischem Material beschickt. Auch die aus dem Vergärungsreaktor kommenden, abgetrennten organischen Feststoffe werden dem Vergaser 12 zur weiteren Verwertung zugeführt.

Das aus der Vergasung resultierende Produktgas wird über eine Transferleitung 19 direkt in die flüssige Phase (Maische) im Vergärungsreaktor geführt. Die Maische wird von dem Produktgas, das ca. 350°C heiß oder optional zur Wärmeauskopplung beispielsweise über einen Wärmetauscher oder über Luftkühlung an der Transferleitung 15 auf ca. 120°C abgekühlt ist, auf bis zu ca. 55°C bzw. 95°C, je nachdem, welche Bakterienstämme bei der Vergärung verwendet werden, erhitzt.

Geeignete thermophile Bakterien (thermophile Archaebakterien bzw. methanogene Archae) sind beispielsweise Thermoactinomyces vulgaris, Thermus aquaticus, Methanobacterium, Thermoautotrophicum, Methanothrix thermophilia u. a.

Geeignete extrem thermophile Bakterien sind Methanococcus, Thermococcus, Methanosarcina, Methanothermus fervidus, Desulfurolobus, Thermotoga, u.a.

Das bei der Vergasung anfallende Produktwasser wird über die Transferleitung 15 zusammen mit dem heißen Produktgas dem Vergärungsreaktor 11 zugeführt und dient zum Anmaischen der flüssigen Phase bei der Vergärung.

Die bei der Vergasung entstehende Asche kann unmittelbar als Rohdünger verwertet oder aber zur Anreicherung des Humus in den Vergärungsreaktor eingetragen werden.

Das erfindungsgemäße, kombinierte Verfahren ist nicht auf bestimmte Typen von Vergasern oder Vergärungsreaktoren beschränkt.

## Patentansprüche

1. Verfahren zur Verwertung von biologischen und anderen organischen Materialien, insbesondere zur Erzeugung von Gas, **dadurch gekennzeichnet, dass** mindestens zwei Biomasseverwertungsverfahren kombiniert werden.

2. Verfahren nach Anspruch 1, **dadurch gekennzeichnet, dass** eine Biomassevergasung mit einer Biomassevergärung kombiniert wird.

3. Verfahren nach Anspruch 2, **dadurch gekennzeichnet, dass** Gas aus der Biomassevergasung in einen Reaktor (1, 11) zur Biomassevergärung gegeben wird.

4. Verfahren nach Anspruch 3, **dadurch gekennzeichnet, dass** zwischen 10 und 40 Vol.-% Produktgas in Bezug auf den gesamten bei der Biomassevergasung und der Biomassevergärung entstehenden Gasmengenstrom dem Biomassevergärungsreaktor (1, 11) zugeführt werden.

5. Verfahren nach Anspruch 3 oder 4, **dadurch gekennzeichnet, dass** das Produktgas vor dem Eintritt in den Biomassevergärungsreaktor (1, 11) durch einen Wärmetauscher geführt wird.

6. Verfahren nach einem der Ansprüche 3 bis 5, **dadurch gekennzeichnet, dass** die Menge und/oder die Temperatur des zugeführten Produktgases eingestellt werden/wird, um die Temperatur bei der Biomassevergasung zu steuern.

7. Verfahren nach einem der Ansprüche 2 bis 6, **dadurch gekennzeichnet, dass** bei der Biomassevergärung thermophile, insbesondere extrem thermophile Bakterien verwendet werden.

8. Verfahren nach einem der Ansprüche 2 bis 7, **dadurch gekennzeichnet, dass** der Biomassevergärung Produktwasser aus der Biomassevergasung zugeführt wird.

9. Verfahren nach einem der Ansprüche 2 bis 8, **dadurch gekennzeichnet, dass** Flugstaub und/oder Asche aus dem Vergasungsreaktor (2, 12) der Vergärung zugeführt werden/wird.

10. Verfahren nach einem der Ansprüche 2 bis 9, **dadurch gekennzeichnet, dass** feste Reststoffe, insbesondere Restholz und Humus, aus der Biomassevergärung entnommen werden, Humus davon abgetrennt und der verbleibende Rest der Biomassevergasung zugeführt wird.

11. Verfahren nach einem der Ansprüche 2 bis 10, **dadurch gekennzeichnet, dass** mit dem Gas ein Gasmotor (5, 17) und/oder eine Gasturbine angetrieben werden/wird.

12. Verfahren nach Anspruch 11, **dadurch gekennzeichnet, dass** hinter dem Gasmotor (5, 17) bzw. der Gasturbine Abgaswärme über einen Wärmetauscher gewonnen und der Biomassevergärung zugeführt wird.
